Europäisches Patentamt

European Patent Office

Office européen des brevets

(19)

(11) Publication number: **0 315 290 B1**

(12) **EUROPEAN PATENT SPECIFICATION**

(45) Date of publication of patent specification: **22.07.92**  (51) Int. Cl.<sup>5</sup>: **A61B 17/32**

(21) Application number: **88303865.5**

(22) Date of filing: **28.04.88**

(54) Atherectomy catheter.

(30) Priority: **23.10.87 US 111715**

(43) Date of publication of application:
**10.05.89 Bulletin 89/19**

(45) Publication of the grant of the patent:
**22.07.92 Bulletin 92/30**

(84) Designated Contracting States:
**CH DE ES FR GB IT LI SE**

(56) References cited:
**WO-A-81/01363**
**US-A- 4 273 128**
**US-A- 4 679 557**

(73) Proprietor: **SCHNEIDER (USA) INC., a Pfizer Company**
**2905 Northwest Boulevard**
**Minneapolis, Minnesota(US)**

(72) Inventor: **Rydell, Mark Anders**
**516 Turnpike Road**
**Golden Valley Minnesota(US)**

(74) Representative: **Boydell, John Christopher et al**
**Stevens, Hewlett & Perkins 1 Serjeants' Inn Fleet Street**
**London EC4Y 1LL(GB)**

EP 0 315 290 B1

## Description

This invention relates generally to intravascular catheters, and more particularly to the design of an atherectomy catheter useful in restoring patency to a blood vessel that is blocked or partially blocked by atheromas or other form of stenotic or thrombotic lesion.

The build-up of atheromas or the formation of thrombi in a blood vessel can cause serious circulatory problems and when complete blockages occur, distal tissues may be deprived of oxygen and nutrients leading to death of those cells distally of the blockage. Thus, the formation of an atheroma in a coronary artery can lead to a coronary infarction, especially when the artery becomes so narrowed by the plaque build-up that a tiny clot or thrombus cannot pass. Similarly, an atheroma or other type of stenotic lesion in a peripheral vein or artery can have a corresponding affect on tissue and cells supplied by the blocked blood vessel.

The treatment of such a condition naturally depends upon the location or site of the blockage. In the case of a blocked or partially blocked coronary artery, it has been the practice to conduct open-heart surgery wherein the blocked vessel is by-passed with an autograft. Similarly, blood vessel shunts have been installed in other body areas as well. Such surgery, however, tends to be quite traumatic involving opening the patient's chest and pericardium in the case of coronary by-pass surgery or extensive excision and vessel replacement in the case of other peripheral blockages.

More recently, following the technique credited to A. Grunzig, a balloon catheter may be used to restore patency to a blood vessel without extensive surgery. A catheter having a small inflatable balloon on its distal end may be routed through the vascular system to the site of the constriction or blockage and when the deflated balloon is appropriately positioned to span the blockage, a fluid may be introduced into the proximal end of the catheter to inflate the balloon to a sufficiently high pressure whereby the blockage may be spread open and patency restored.

As is pointed out in the Auth U.S. Patent 4,445,509, there are certain deficiencies in the Grunzig procedure which render it ineffective in certain applications. For example, the blockage may be such that it is now possible to safely force the distal tip of the catheter through the blockage prior to the inflation of the balloon. In such a situation it would be desirable if one could safely "tunnel" through the blockage using an appropriate cutting tool. Once a passage has been formed during such tunnelling operation, a balloon can be advanced into the occlusion until it is totally across it. Once so positioned, the balloon can then be identified and the angioplasty procedure completed.

WO-A-81 1363 discloses a surgical device for cutting and removing tissue with a high degree of accuracy from locations which would otherwise be inaccessible. The device comprises a pair of coaxial tubes which may be rotated relative to one another, each of said tubes having respective end walls in which are formed apertures. The arrangement is such that, as the tubes are rotated relative to one another, the two apertures move in and out of registry with one another, thus resulting in a cutting action. Suction through the inner tube removes debris resulting from the cutting operation.

In accordance with the present invention, there is provided an atherectomy catheter device having an outer elongate flexible tubular member having a proximal end and a distal end, said distal end being of an outside diameter which is smaller than the outside diameter of the remaining portion of said outer tubular member,

an inner elongate flexible tubular member coaxially disposed within said outer tubular member and having a proximal end and a distal end with the outside diameter of said inner tubular member being generally equal to the inside diameter of said outer tubular member at said distal end of said outer tubular member;

drive means disposed at the proximal ends of said outer and inner tubular members for rotating said inner tubular member relative to said outer tubular member;

said device being characterised in that said distal end of said inner tubular member extends out beyond the distal end of said outer tubular member;

in that a cutter member is affixed to said distal end of said inner tubular member, said cutter member having a tubular portion surrounding said distal end of said outer tubular member and a dome-shaped distal end portion, the outside diameter of said tubular portion of said cutter member being generally equal to the outside diameter of said remaining portion of said outer tubular member,

and in that a plurality of openings is formed through said dome-shaped portion and in fluid communication with the lumen of said inner tubular member.

Preferably the drive means which is configured to rotate the inner tubular member relative to the outer tubular member, is further operable to allow the simultaneous perfusion of a liquid through the outer tubular member and the aspiration of fluids through the lumen of the inner tubular member. In this fashion, upon insertion of the catheter within the patient's vascular system and advancing the distal tip portion thereof to the site of the lesion to be excised, the site can be flooded with a flushing liquid and when the cutter is driven at high speed and advanced into the lesion, the fluids and debris sectioned from the lesion can be aspirated through the lumen of the inner tubular member and collected in a suitable vessel at the proximal end of the assembly. By driving the cutter at a relatively high speed, e.g. 30,000 rpm, the cutter, as configured, finely divides the fatty lesion and effectively liquifies the material prior to its being aspirated back through the lumen of the cutter tube.

It is accordingly a principal object of the present invention to provide a catheter assembly including an outer elongated flexible tubular member and a concentrically disposed, loosely fitting elongated inner tubular member, the inner tubular member being provided with a cutter attachment at its distal end and adapted to be driven by a drive means disposed at the proximal end of the catheter assembly.

Another object of the invention is to provide an atherectomy catheter in which a rotatable cutter is disposed at the distal end thereof and means are provided at the proximal end for driving the cutter at the high rotational speed while simultaneously injecting a flushing liquid and aspirating the treatment site.

These and other objects and advantages of the invention will become apparent to those skilled in the art from the following detailed description of a preferred embodiment, especially when considered in conjunction with the accompanying drawings in which like numerals in the several views refer to corresponding parts.

Figure 1 is a partially sectioned view illustrating a preferred embodiment of the present invention;

Figure 2 is a greatly enlarged view of one cutter head arrangement;

Figure 3 is an end view of the cutter head of Figure 2; and

Figure 4 is a greatly enlarged side elevation of an alternative cutter head arrangement.

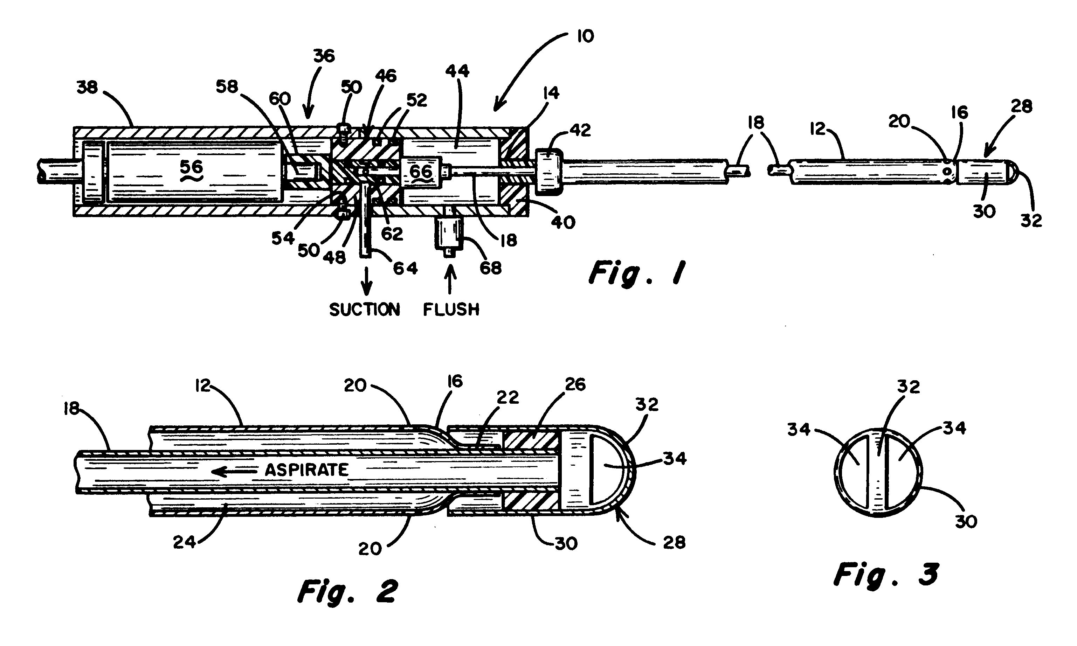

Referring to Figure 1, the surgical device of the present invention is seen to comprise an atherectomy catheter which is indicated generally by numeral 10. It includes an outer elongated, flexible tubular member 12 having a proximal end 14 and a distal end 16. Disposed within the lumen of the outer tubular member 12 is an elongated flexible inner tubular member 18 which extends the full length of the outer tubular member.

Referring to Figure 2, it can be seen that near the distal end 16 of the outer tube, a series of ports 20 are formed through the wall thereof and it tapers down to a distal end portion 22 which is of a lesser outside diameter than the remaining portion of the catheter body 12. Thus, an annular space 24 is created between the outside diameter of the inner tube 18 and the inside diameter of the outer tube 12. As will be illustrated subsequently, when a liquid is appropriately injected at the proximal end of the catheter assembly, it will profuse through this flush lumen 24 and exit the ports 20.

The outer tubular member in zone 22 conforms to the outside dimension of the inner tubular member 18 and provides a bearing surface for the inner tubular member 18 as it is driven. Adhesively joined to the portion of the inner tubular member 18 which extends distally beyond the end of the outer tubular member 12 is an annular spacer 26 and bonded to the spacer 26 is a cutter head 28.

With reference to Figures 1 and 3, one style of cutter head is seen to comprise a generally tubular portion 30, which at least partially surrounds the distal end 16 of the outer tubular member 12, and a radially disposed arcuate loop 32 which projects longitudinally outward from the distal end of the tubular portion 30 of the cutter head 28. The loop effectively divides the open end of the tubular cutter member into a pair of openings 34 through which blood, a flushing liquid, and any tissue debris may be drawn when a vacuum or negative pressure is suitably applied to the proximal end of the inner tubular member 18.

The outer tubular member 12 is preferably extruded from a plastic selected from the group including polyester, nylon and polyolefin. The inner tubular member 18 may also be formed with a like plastic, but with polyester being preferred. The cutter member 28 can be fabricated from either metal or plastic and the overall outside diameter thereof, as well as that of the outer tubular member 12, is determined by the particular location of the atheroma to be treated and the size of the blood vessels leading thereto.

Connected to the proximal end of the outer tubular member is a drive means 36 which, among other functions, is used to rotate the inner tubular member within the lumen of the outer tubular member 12. With reference to Figure 1, it is seen to include a rigid tubular housing 38 having a plug 40 fitted into the distal end thereof. Extending through a bore formed in the plug 40 is a fitting 42, preferably of the compression type, for joining the outer tubular member 12 to the interior chamber 44 of the tubular housing 38.

Disposed within the housing 38 is a rotary union, indicated generally by numeral 46. It comprises an outer tubular sleeve member 48 fastened to the housing 38 by pins 50. These pins preclude relative rotation between the tubular sleeve 48 and the housing 38. To prevent the flushing liquid from passing from the chamber 44 beyond the rotary union 46, O-rings, as at 52, are disposed in annular grooves spanning the tubular sleeve 48. The rotary union further comprises a hollow manifold member 54 which fits within the bore of the outer tubular sleeve 48. This manifold member is dimensioned so as to rotate within that bore when driven by a motor means. In the preferred embodiment, an air motor of the type found in dental drills or the like may be used. Typically, such motors may be designed to rotate at very high speeds, e.g., 30,000 rpm. The shaft 58 of that motor is keyed in a coupling 60 connected to the proximal end of the hollow manifold member 54.

Formed between the outer tubular sleeve 48 and the manifold member contained therein is an annular recess 62 which is ported to the interior of the hollow manifold member. Furthermore, a tubular fitting 64 passes through a bore formed radially through the side wall of the rigid tubular housing 38 and through the wall of the outer tubular sleeve 48 to communicate with that recess 62. The rotatable hollow manifold 54 is also joined to the proximal end of the inner elongated flexible tubular member 18 by a suitable coupler 66.

In use, the elongated catheter assembly is appropriately introduced into the vascular system and advanced until the cutter tip 32 is positioned closely adjacent to the atheroma or other lesion to be excised. Next, a flushing liquid may be introduced through the fitting 68 and into the chamber 44 of the drive means 36. From there, the liquid flows through the lumen of the outer tubular member 12 between its inner wall and the outer wall of the inner tubular member 18. The liquid then exits the ports 20 formed through the side wall of the outer tubular member near its distal end. When a suitable source of negative pressure is applied to the suction fitting 64, the flush liquid along with blood and/or tissue debris, which may be filed from the atheroma, is drawn through the openings 34 formed in the distal end of the cutter 30, through the lumen of the inner tubular member or drive tube 18, and thence through the coupler 66 and into the hollow manifold member 54. The fluid then flows through a port communicating with the annular recess 62 and then out the suction fitting 64 into a suitable receptacle (not shown). At the same time, when the motor means 56 is energized, it drives the hollow manifold member of the rotary union within its tubular sleeve and the drive tube 18 coupled thereto by coupler 66. When the catheter is advanced, and modest pressure is applied between the cutter loop 32 and the tissue being excised, that tissue is finally divided by the rapidly spinning cutter load and washed by blood and flushing liquid through the central lumen of the tube 18 and into the collecting receptacle connected to the suction fitting 64. Once the atheroma has been completely penetrated, blood flow through the blood vessel is restored.

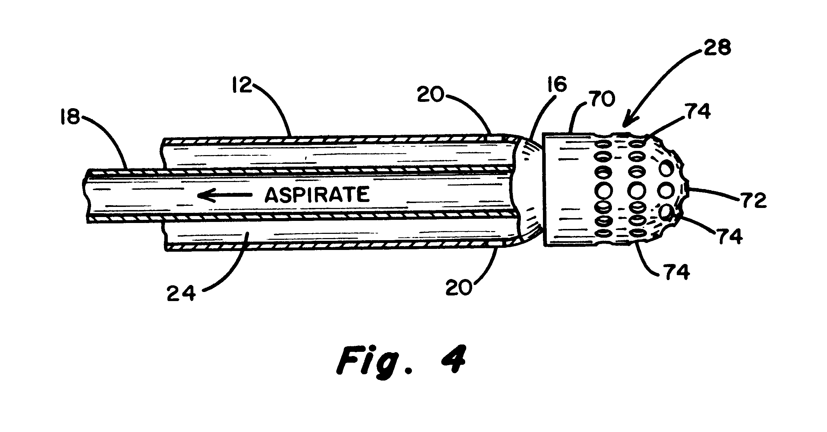

Referring to Figure 4, there is shown a side elevation of an alternative design for the cutter head 28. As in the embodiment of Figures 1 through 3, the cutter head is attached to the distal end of drive tube 18 for rotation therewith. Rather than having a cutting loop 32 effectively defining two aspiration openings 34, the cutting head 28 of Figure 4 comprises a hollow, bullet-shaped, thin wall sleeve 70 having a hemispherical shaped distal end 72. With no limitation intended, the sleeve 70 may have a thickness of 0.005 inches and a dome radius of 0.50 inches. It may have an overall length on the order of 0.125 inches and can be secured to the end of tube 18 in the same manner as cutter head 30 of Figure 2.

Formed through the hemispherical end portion of the cutter head of Figure 4 is a pattern of holes 74. The number of and size of the holes 74 is preferably such that their total area is generally equal to the cross-sectional area of the lumen of the drive tube 18. Also, the diameter of the holes 74 should be two to three times the thickness of the wall of the dome portion 72. For a dome thickness of 0.005 inches, approximately 42 holes, each 0.015 inches in diameter has been found to produce excellent results. Holes 42 are preferably formed in a laser drilling operation. In use, when the drive tube is rotated as previously described, and the cutter head of Figure 4 is advanced against the atheroma to be excised, the cutter head acts like a grater to finely divide the fatty tissue of the atheroma so that the debris, along with flush liquid and body fluids can be aspirated back through the cutter openings and the lumen of the inner tube 18 to the collection chamber (not shown).

## Claims

1. An atherectomy catheter device (10) having an outer elongate flexible tubular member (12) having a proximal end (14) and a distal end (16), said distal end being of an outside diameter which is smaller than the outside diameter of the remaining portion of said outer tubular member,

an inner elongate flexible tubular member (18) coaxially disposed within said outer tubular member and having a proximal end and a distal end with the outside diameter of said inner tubular member being generally equal to the inside diameter of said outer tubular member at said distal end of said outer tubular member;

drive means (36) disposed at the proximal ends of said outer and inner tubular members for rotating said inner tubular member relative to said outer tubular member;

said device being characterised in that said distal end of said inner tubular member extends out beyond the distal end of said outer tubular member;

in that a cutter member (28) is affixed to said distal end of said inner tubular member, said cutter member having a tubular portion (30) surrounding said distal end of said outer tubular member and a dome-shaped distal end portion (32), the outside diameter of said tubular portion of said cutter member being generally equal to the outside diameter of said remaining portion of said outer tubular member,

and in that a plurality of openings (34) is formed through said dome-shaped portion and in fluid communication with the lumen of said inner tubular member.

2. The catheter device as in claim 1 in which said outer tubular member (12) has at least one orifice (20) extending through its side wall a predetermined distance proximal to said distal end (16) of said outer tubular member.

3. The catheter device as in Claim 2 and further characterised by means (68, 44, 42) associated with said drive means (36) for injecting a liquid between the walls of said inner and outer tubular members, said liquid exiting the lumen of said outer tubular member through said at least one orifice (20).

4. The catheter device as in Claim 1 further characterized by means associated with said drive means (36) for aspirating fluids through said plurality of openings (34) in said distal end portion of said cutter member (28), through the lumen of said inner tubular member (18) and out the proximal end of said inner tubular member as said inner tubular member is rotated.

5. The catheter device as in Claim 1 in which said drive means (36) is characterized by,
   (a) a rigid tubular housing (38) having a plug (40) disposed in the distal end thereof;
   (b) a fitting (42) extending through said plug and joined to said proximal end (14) of said outer tubular member (12);
   (c) a rotary union (46) including
      (i) an outer tubular sleeve (48) keyed to and sealingly fitted within said rigid tubular housing;
      (ii) a hollow manifold member (54) journaled for rotation within said outer tubular sleeve and including an annular recess (62) formed between said manifold member and said tubular sleeve, said recess being in fluid communication with the interior of said hollow manifold;
   (d) means (66) coupling said manifold member to said proximal end of said inner tubular member.
   (e) motor means contained within said rigid tubular housing, the shaft of said motor means coupled to said manifold member to rotate same within said outer tubular sleeve; and
   (f) a fluid port (64) extending through said housing (38) and said tubular sleeve (48) communicating with said annular recess (62).

6. The catheter device as in Claim 5 in which said motor means is air driven.

7. The catheter device as in Claim 1 and further characterized by an annular spacer (26) joining said distal end of said inner tubular member (18) to said tubular portion (30) of said cutter member (28).

8. The catheter device as in Claim 1 in which said plurality of openings (34) in said dome-shaped distal end portion (32) of said cutter member (28) define a radially disposed arcuate loop projecting longitudinally outward from the distal end of said tubular portion of said cutter member.

9. The catheter device as in Claim 1 in which the total area of said plurality of openings (34) is generally equal to or greater than the cross-sectional area of the lumen of said inner tubular member.

**Revendications**

1. Instrument (10) à cathéter d'athérectomie comprenant un élément tubulaire extérieur allongé souple (12) ayant une extrémité proximale (14) et une extrémité distale (16), ladite extrémité distale ayant un diamètre extérieur qui est plus petit que le diamètre extérieur du reste dudit élément tubulaire extérieur,

un élément tubulaire allongé intérieur souple (18) disposé coaxialement à l'intérieur dudit élément tubulaire extérieur et ayant une extrémité proximale et une extrémité distale, le diamètre extérieur dudit élément tubulaire intérieur étant sensiblement égal au diamètre intérieur dudit élément tubulaire extérieur à ladite extrémité distale dudit élément tubulaire extérieur ;

des moyens d'entraînement (36) disposés aux extrémités proximales desdits éléments tubulaires extérieur et intérieur et destinés à faire tourner ledit élément tubulaire intérieur par rapport audit élément tubulaire extérieur ;

ledit instrument étant caractérisé en ce que ladite extrémité distale dudit élément tubulaire intérieur se prolonge au-delà de l'extrémité distale dudit élément tubulaire extérieur ;

en ce qu'un élément de coupe (28) est fixé à ladite extrémité distale dudit élément tubulaire intérieur, ledit élément de coupe comportant une partie tubulaire (30) entourant ladite extrémité distale dudit élément tubulaire extérieur et une partie d'extrémité distale (32) en forme de dôme, le diamètre extérieur de ladite partie tubulaire dudit élément de coupe étant sensiblement égal au diamètre extérieur dudit reste dudit élément tubulaire extérieur,

et en ce que de multiples trous (34) sont réalisés à travers ladite partie en forme de dôme et sont en communication fluidique avec le passage dudit élément tubulaire intérieur.

2. Instrument à cathéter selon la revendication 1, dans lequel ledit élément tubulaire extérieur (12) comporte au moins un orifice (20) traversant sa paroi latérale à une distance prédéterminée, dans le sens proximal, de ladite extrémité distale (16) dudit élément tubulaire extérieur.

3. Instrument à cathéter selon la revendication 2, caractérisé par ailleurs par des moyens (68, 44, 42) associés auxdits moyens d'entraînement (36) pour injecter un liquide entre les parois desdits éléments tubulaires intérieur et extérieur, ledit liquide sortant du passage dudit élément tubulaire extérieur par ledit au moins un orifice (20).

4. Instrument à cathéter selon la revendication 1, caractérisé par ailleurs par des moyens associés auxdits moyens d'entraînement (36) pour aspirer des fluides par lesdits multiples trous (34) de ladite partie extrême distale dudit élément de coupe (28), par le passage dudit élément tubulaire intérieur (18) et vers l'extérieur de l'extrémité proximale dudit élément tubulaire intérieur lorsque ledit élément tubulaire intérieur est entraîné en rotation.

5. Instrument à cathéter selon la revendication 1, dans lequel lesdits moyens d'entraînement (36) sont caractérisés par

(a) une enveloppe tubulaire rigide (38) comportant un bouchon (40) disposé dans son extrémité distale ;

(b) un raccord (42) passant à travers ledit bouchon et relié à ladite extrémité proximale (14) dudit élément tubulaire extérieur (12) ;

(c) un joint rotatif (46) comprenant

(i) un manchon tubulaire extérieur (48) claveté sur et ajusté de manière étanche à l'intérieur de ladite enveloppe tubulaire rigide ;

(ii) un élément collecteur creux (54) monté rotatif à l'intérieur dudit manchon tubulaire extérieur et renfermant une cavité annulaire (62) formée entre ledit élément collecteur et ledit manchon tubulaire, ladite cavité étant en communication fluidique avec l'intérieur dudit collecteur creux ;

(d) des moyens (66) pour accoupler ledit élément collecteur à ladite extrémité proximale dudit élément tubulaire intérieur,

(e) un moteur logé à l'intérieur de ladite enveloppe tubulaire rigide, l'arbre dudit moteur étant accouplé audit élément collecteur de manière qu'il fasse tourner ce dernier à l'intérieur dudit manchon tubulaire extérieur ; et

(f) un orifice (64) pour fluide qui traverse ladite enveloppe (38) et ledit manchon tubulaire (48) et qui communique avec ladite cavité annulaire (62).

6. Instrument à cathéter selon la revendication 5, dans lequel ledit moteur est à entraînement pneumatique.

7. Instrument à cathéter selon la revendication 1, caractérisé par ailleurs par une entretoise annulaire (26) reliant ladite extrémité distale dudit élément tubulaire intérieur (18) et ladite partie tubulaire (30) dudit élément de coupe (28).

8. Instrument à cathéter selon la revendication 1, dans lequel lesdits multiples trous (34) de ladite partie d'extrémité distale (32) en forme de dôme dudit élément de coupe (28) délimitent un arceau incurvé, disposé radialement, saillant longitudinalement vers l'extérieur de ladite extrémité distale de ladite partie tubulaire dudit élément de coupe.

9. Instrument à cathéter selon la revendication 1, dans lequel la superficie totale desdits multiples trous (34) est sensiblement égale ou supérieure à la superficie transversale du passage dudit élément tubulaire intérieur.

**Patentansprüche**

1. Atherektomie-Katheter-Vorrichtung (10) mit einem äußeren langen flexiblen röhrenförmigen Teil (12) mit einem proximalen Ende (14) und einem distalen Ende (16), wobei das distale Ende einen Außendurchmesser hat, der schmaler ist als der Außendurchmesser des restlichen Teils des äußeren röhrenförmigen Teils,

einem inneren langen flexiblen röhrenförmigen Teil (18), der koaxial innerhalb des äußeren röhrenförmigen Teils angeordnet ist, und ein proximales Ende und ein distales Ende hat, wobei der Außendurchmesser des inneren röhrenförmigen Teils im allgemeinen gleich ist zum Innendurchmesser des äußeren röhrenförmigen Teils am distalen Ende des äußeren röhrenförmigen Teils;

einem Antriebsmittel (36), das an den proximalen Enden der äußeren und inneren röhrenförmigen Teile zum Drehen des inneren röhrenförmigen Teils relativ zum äußeren röhrenförmigen Teil angeordnet ist;

wobei die Vorrichtung dadurch gekennzeichnet ist, daß sich das distale Ende des inneren röhrenförmigen Teils über das distale Ende des äußeren röhrenförmigen Teils hinaus erstreckt;

ein Schneide-Element (28) am distalen Ende des inneren röhrenförmigen Teils befestigt ist, wobei das Schneide-Element einen röhrenförmigen Teil (30) aufweist, der das distale Ende des äußeren röhrenförmigen Teils umgibt, und einen kuppelförmigen Teil (32) am distalen Endteil, wobei der Außendurchmesser des röhrenförmigen Teils des Schneide-Elements im allgemeinen gleich ist zum Außendurchmesser des restlichen Teils des äußeren röhrenförmigen Teils;

und daß eine Vielzahl von Öffnungen (34) durch den kuppelförmigen Teil ausgebildet sind, die in Fluid-Verbindung mit dem Lumen des inneren röhrenförmigen Teils stehen.

2. Katheter-Vorrichtung nach Anspruch 1, bei welcher der äußere röhrenförmige Teil (12) mindestens eine Öffnung (20) aufweist, die sich durch seine Seitenwandung in einem vorbestimmten Abstand proximal zum distalen Ende (16) des äußeren röhrenförmigen Teils erstreckt.

3. Katheter-Vorrichtung nach Anspruch 2, außerdem gekennzeichnet durch Mittel (68, 44, 42), die mit dem Antriebsmittel (36) zum Injizieren einer Flüssigkeit zwischen die Wandungen der inneren und äußeren röhrenförmigen Teile verbunden sind, wobei die Flüssigkeit das Lumen des äußeren röhrenförmigen Teils durch die mindestens eine Öffnung (20) verläßt.

4. Katheter-Vorrichtung nach Anspruch 1, außerdem gekennzeichnet durch Mittel, die mit dem Antriebsmittel (36) zum Absaugen von Fluiden durch die Vielzahl von Öffnungen (34) im distalen Endteil des Schneide-Elements (28) durch das Lumen des inneren röhrenförmigen Teils (18) und aus dem proximalen Ende des inneren röhrenförmigen Teils, wenn der innere röhrenförmige Teil gedreht wird, verbunden sind.

5. Katheter-Vorrichtung nach Anspruch 1, bei der das Antriebsmittel (36) gekennzeichnet ist durch

(a) ein stabiles röhrenförmiges Gehäuse (38) mit einem Stecker (40), der an dessen distalem Ende angeordnet ist;

(b) ein Paßstück (42), das sich durch den Stecker erstreckt und mit dem proximalen Ende (14) des äußeren ringförmigen Teils (12) verbunden ist;

(c) eine Drehvorrichtung (46), mit

(i) einer äußeren röhrenförmigen Buchse (48), die in dem stabilen röhrenförmigen Gehäuse befestigt und dicht darin eingepaßt ist;

(ii) einem hohlen Verteilerteil (54), das zur Drehung innerhalb der äußeren röhrenförmigen Buchse angeordnet ist, und einer ringförmigen Ausnehmung (62), die zwischen dem Verteilerteil und der röhrenförmigen Buchse ausgebildet ist, wobei die Ausnehmung in Fluid-Kommunikation mit dem Inneren des hohlen Verteilerteils steht;

(d) ein Mittel (66) zur Kupplung des Verteilerteils an das proximale Ende des inneren röhrenförmigen Teils;

(e) eine Motor-Einrichtung, die in dem stabilen röhrenförmigen Gehäuse enthalten ist, wobei die Wellenachse der Motor-Einrichtung an das Verteilerteil gekuppelt ist, um dieses in der äußeren röhrenförmigen Buchse zu drehen; und

(f) eine Fluid-Öffnung (64), die sich durch das Gehäuse (38) und die röhrenförmige Buchse (48) erstreckt und mit der ringförmigen Ausnehmung (62) kommuniziert.

6. Katheter-Vorrichtung nach Anspruch 5, bei der die Motor-Einrichtung luftgetrieben ist.

7. Katheter-Vorrichtung nach Anspruch 1 und außerdem gekennzeichnet durch ein ringförmiges Distanzstück (26), welches das distale Ende des inneren röhrenförmigen Teils (18) mit dem röhrenförmigen Teil (30) des Schneide-Elements (28) verbindet.

8. Katheter-Vorrichtung nach Anspruch 1, bei der die Vielzahl von Öffnungen (34) im kuppelförmigen distalen Endteil (32) des Schneide-Elements (28) eine radial angeordnete bogenförmige Schleife definieren, die vom distalen Ende des röhrenförmigen Teils des Schneide-Elements longitudinal nach außen vorspringt.

9. Katheter-Vorrichtung nach Anspruch 1, bei der die Gesamtfläche der Vielzahl von Öffnungen (34) im allgemeinen gleich oder größer ist als die Querschnittsfläche des Lumens des inneren röhrenförmigen Teils.

Fig. 1

Fig. 2

Fig. 3

ASPIRATE

SUCTION    FLUSH

Fig. 4